# EUROPEAN PATENT SPECIFICATION

(11) **EP 1 414 465 B1**
(45) Date of publication and mention of the grant of the patent: **13.09.2006**
(21) Application number: 01987667.1
(22) Date of filing: 17.10.2001
(51) Int. Cl.: A61K 31/56, A61P 35/00

(54) **USE OF INHIBITORS OF PROGESTERONE RECEPTOR FOR TREATING STEROID RESISTANT BREAST CANCER**
VERWENDUNG VON PROGESTERONREZEPTORHEMMERN ZUR BEHANDLUNG VON STEROID-RESISTENTEM BRUSTKREBS
UTILISATION D'INHIBITEURS DU PROGESTERONE POUR LE TRAITEMENT DU CANCER DU SEIN RESISTANT AUX STEROIDS

(30) Priority: 18.10.2000 DE 10051609; 18.10.2000 US 241010 P
(43) Date of publication of application: 06.05.2004
(73) Proprietor: Schering Aktiengesellschaft, 13353 Berlin (DE)
(72) Inventor: LICHTNER, Rosemarie, 10823 Berlin (DE); FUHRMANN, Ulrike, 10587 Berlin (DE)
(86) International application number: PCT/EP2001/012004
(87) International publication number: WO 2002/032429

(56) References cited:
- EP-A- 0 466 315
- WO-A-00/46234
- WO-A-98/34947
- WO-A-99/33855
- US-A- 5 238 950

## Description

The invention relates to the use of progesterone receptor inhibitors for the manufacture of a medicament for the treatment of steroid resistant breast cancer.

Estradiol and progesterone are involved in the development of breast cancer. At the time of diagnosis, however, only about 1/3 of the tumors show a steroid hormone dependency. It is assumed that in the majority of steroid hormone-resistant tumors, the proliferation control for local-acting autocrine or paracrine peptidic growth factors is taken over. In this case, invasive tumors with extremely poor prognosis that are growth-factor-receptor-positive and steroid hormone-resistant result (Elledge et al., Semin. Onkol. 19 (1992), 244-253).

Growth factors regulate the cell growth by activation of intracellular signal transduction pathways after binding to highly affine tyrosine kinase receptors on the cell surface. More recent findings suggest that breast carcinoma cells can be sensitized by progestins for the mitogenic action of EGF (Groshong et al., Mol. Endocrinol. 11 (1997), 1593-1607). Thus, for example, it was possible for progesterone in the human breast carcinoma cell line T47D to induce the onset of cells in the S-phase accompanied by a transient increase of the activity of cyclin 1D and the cyclin-dependent kinase 4. The growth stimulation is limited to a single cycle, however, and is followed by a growth arrest at the G1/S-transition of the second cycle (Groshong et al. (1997), supra: Musgrove et al., Mol. Cell. Biol. 13 (1993), 3577-3587). In its condition that is stopped by progesterone, the cells are sensitive to the proliferative action of EGF. In addition, it was shown that progesterone enhances the action of EGF on T47D cells by ramping up EGFR, Erb2 and Erb3 and increases the tyrosine phosphorylation of signal molecules (Lange et al., J. Biol. Chem. 273 (1998), 31308-31316; Richer et al., J. Biol, Chem. 273 (1998), 31317-31326). In contrast, it has not yet been possible to show an inhibition of the action of EGF on tumor cells by influencing the progesterone receptor.

Within the scope of tests leading to this invention, it has now been found, surprisingly enough, that inhibitors of the progesterone receptor, e.g., 17α-fluoroalkyl steroids, can at least partially inhibit the binding of growth factors, such as EGF, to breast carcinoma cells.

A subject of this invention is thus the use of an inhibitor of the progesterone receptor for the manufacture of a medicament for the treatment of steroid resistant breast cancer. An inhibitor of the progesterone receptor in terms of this invention is preferably a substance that competitively inhibits the binding of progesterone to its receptor. In this case, the inhibitor of the progesterone receptor is preferably selected from 17α-fluoroalkyl steroids, as they are disclosed in, e.g., WO98/34947. These 17α-fluoroalkyl steroids exhibit general formula I: 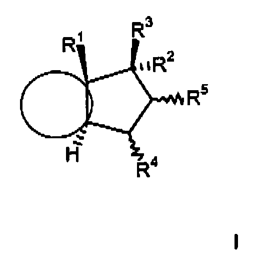 in which
- R¹: stands for a methyl or ethyl group,
- R²: stands for a radical of formula CₙFₘHₒ, whereby n = 2, 3, 4, 5 or 6, m > 1 and m + o = 2n + 1,
- R³: stands for a free, etherified, or esterified hydroxy group,
- R⁴ and R⁵: each stand for a hydrogen atom, together for an additional bond or a methylene group,
- St: stands for a steroidal ABC-ring system of partial formula A, B or C 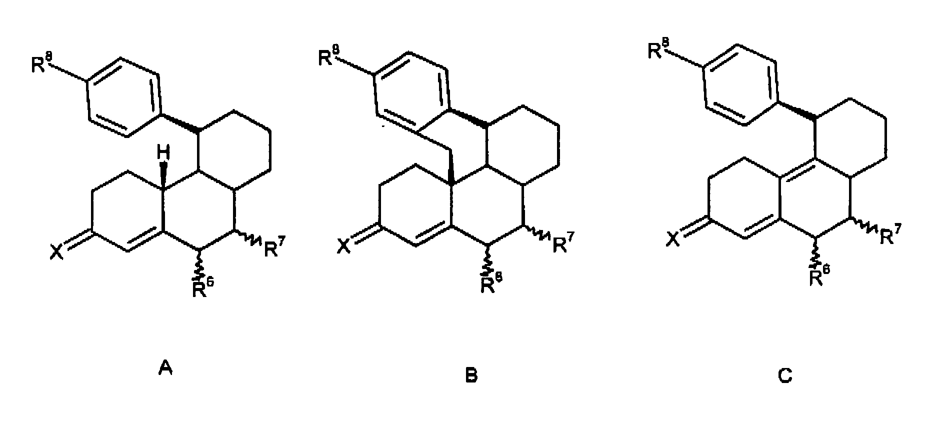
in which
- R⁶: means a hydrogen atom, a straight-chain C₁-C₄ alkyl group or a branched C₃-C₄ alkyl group or a halogen atom,
- R⁷: means a hydrogen atom, a straight-chain C₁-C₄ alkyl group or a branched C₃-C₄ alkyl group, or, if St stands for a steroidal ABC-ring system A or B, in addition R⁶ and R⁷ together mean an additional bond,
- X: means an oxygen atom, a hydroxymino grouping = N - OH or two hydrogen atoms,
- R⁶: means a radical Y or an aryl radical that is optionally substituted with a group Y in several places, whereby Y is a hydrogen atom, a halogen atom, an -OH, -NO₂, -N₃, -CN, -NR^{9a}R^{9b}, -NHSO₂R⁹, -CO₂R⁹, C₁-C₁₀ alkoxy, C₁-C₁₀ alkanoyloxy, benzoyloxy-C₁-C₁₀ alkanoyl, C₁-C₁₀ hydroxyalkyl or benzoyl group,
and R^{9a} and R^{9b} are the same or different and like R⁹ represent a hydrogen atom or a C₁-C₁₀ alkyl group,
and for radicals -NR^{9a}R^{9b}, also their physiologically compatible salts with acids and for radicals -CO₂R⁹ with R⁹ in the meaning of hydrogen also their physiologically compatible salts with bases.

An especially preferred example of such inhibitors of the progesterone receptor is the compound 11β-(4-acetylphenyl)-17β-hydroxy-17α-(1,1,2,2,2-pentafluoroethyl)-estra-4,9-dien-3-one (compound A below).

The action of the progesterone receptor inhibitors is found especially in the case of tumor cells that have a high and/or constitutive progesterone receptor expression, for example the progesterone receptor-positive breast carcinoma cell line T47D (Sartorius et al., Cancer Res. 54 (1994), 3668-3877).

The progesterone receptor inhibitors inhibit the progesterone-induced enhancement of the expression of growth factors, especially those factors that bind to growth factors of the EGF receptor family, such as, for example, the EGF receptor. The inhibitors especially preferably inhibit the binding of EGF to human breast carcinoma cells.

According to this invention, the progesterone receptor inhibitors can therefore be used for therapy of breast carcinoma of steroid-dependent growth to growth-factor-dependent growth in mammals and preferably in humans. In this way, an effective treatment of the tumor can take place in the stage of the steroid-dependent growth, e.g. by antiestrogens, without the tumor being able to progress in the stage of the growth-factor-dependent growth, associated with a considerable worsening of the prognosis for the patient. The administration of the progesterone receptor inhibitors can also produce a slowing of tumor growth in the stage of the growth-factor-dependent growth.

The administration of the progesterone receptor inhibitors can be carried out according to commonly used methods, for example locally, topically, subcutaneously, enterally or parenterally. For enteral administration, especially tablets, coated tablets, capsules, pills, suspensions or solutions are suitable, which can be produced in the usual way with the additives and vehicles that are known in gallenicals. For local or topical use, for example, vaginal suppositories or transdermal systems such as skin patches are suitable. The subcutaneous administration can be carried out by injection with an oily solution.

A dosage unit can contain, for example, 0.1 to 100 mg of active compound(s) (= inhibitor(s) of the progesterone receptor). For administration in humans, the daily dose of the active compound(s) is 0.1 to 400 mg, preferably 10-100 mg and especially 50 mg.

In addition, the invention is to be explained by the following examples and figures. It is noted that the compounds hydroxytamoxifen, ZM 182 780, estradiol, ZK 191 703, onapristone, and RU486 within the following examples and figures are not comprised by the present invention, respectively do not fall under formula I. These compounds are only used as comparative examples.

Here:
- Figure 1: shows the antiproliferative action of test substances on the breast carcinoma cell line T47D.
- Figure 2: shows the amounts of protein of progesterone receptor (PR) and estrogen receptor (ER) in breast carcinoma cell line T47D.
- Figure 3: shows the transcriptional activity of the progesterone receptor in T47D cells.
- Figure 4: shows a Scatchard analysis of the binding of EGF to T47D cells as a function of the presence of test substances.
- Figure 5: shows the dependence of the binding of EGF to T47D cells on the presence of test substances.

### Example

### 1. Materials and Methods

### Materials:

¹²⁵I-EGF (100 mCi/mmol) was obtained by Amersham Buchler. Compound A, hydrotamoxifen (4-OH-Tam), ZM182780 and estradiol were synthesized in the Institut für Arzneimittelchemie [Institute for Pharmaceutical Agent Chemistry] of the Schering AG according to known methods.

### Cell lines:

The human estrogen receptor (ER)- and progesterone receptor (PR)-positive breast carcinoma cell line T47D (Freake et al., BBRC 101 (1981), 1131-1138) was used.

### Growth studies:

The tumor cells were cultivated at 5000 cells/well in 96-well plates for 6 days in RPMI medium plus 10% bovine serum, 200 nM of insulin and 0.1 nM of estradiol in the presence of the compounds that are indicated in each case, and the growth was determined by staining with crystal violet.

### Amount of PR and ER protein:

The amounts of PR and ER in cell lysates are determined with use of steroid binding assays with radiolabeled progesterone or estradiol according to methods described in Fuhrmann et al. (Contraception 54 (1996), 243-251).

### Binding of ¹²⁵I-EGF to tumor cells:

R5020-Pretreated T47D cells were incubated for 2 hours with ¹²⁵I-EGF at 4°C. The unspecific binding was always less than 10% of the total binding.

### Transactivation assay:

T47D cells were transiently transfixed with MTV-LUC (Cato et al., EMBO J., 9: 2237-40) and cultivated in the absence or the presence of 1 nM of R5020. In the test on a PR-mediated antagonism, the transiently transfixed T47D cells were treated with R5020 and in addition with increasing concentrations of compound A or RU486. After 24 hours, a luciferase test was performed.

### 2. Results

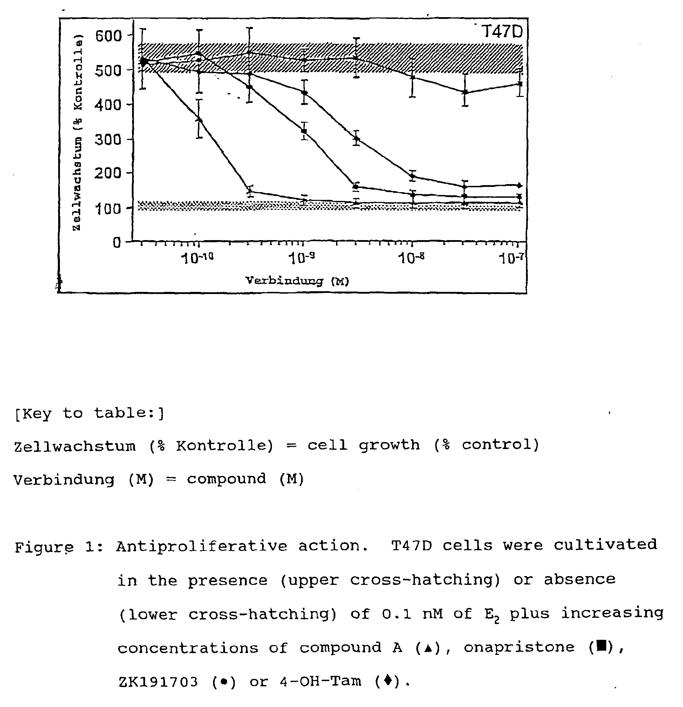

Figure 1 shows the antiproliferative action of various test substances. T47D cells were cultivated in the presence (upper cross-hatching) or absence (lower cross-hatching) of 0.1 nM of E₂ plus increasing concentrations of compound A (▲), onapristone (■), ZK191703 (●) or 4-OH-Tam (◆). In the case of T47D cells, compound A also shows a significant antiproliferative action at extremely small concentrations.

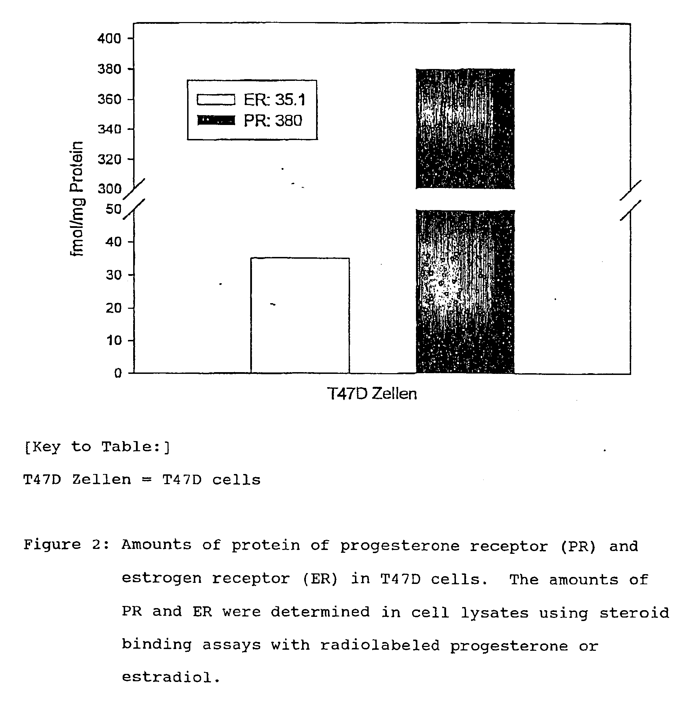

Figure 2 shows the amounts of PR- and ER protein in T47D cells.

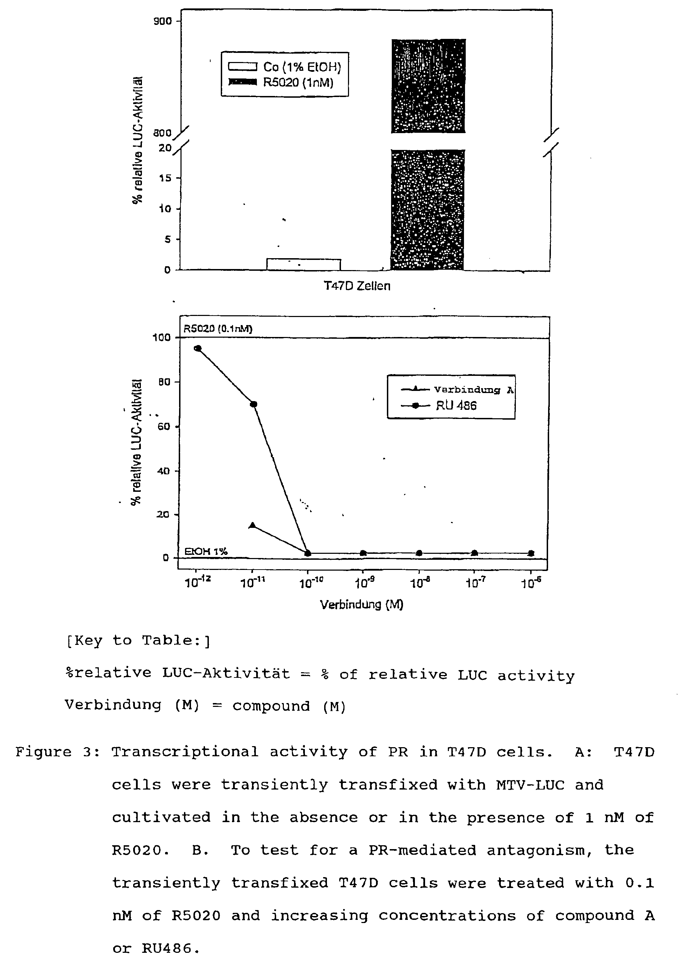

Figure 3 shows the transcriptional activity of PR in T47D cells, whereby the respective cells were transiently transfixed with MTV-LUC and cultivated (a) in the absence of (Co) or the presence of 1 nM of R5020. In the test for a PR-mediated antagonism, the transiently transfixed T47D cells were treated with 0.1 nM of R5020 and increasing concentrations of compound A or RU468 (b).

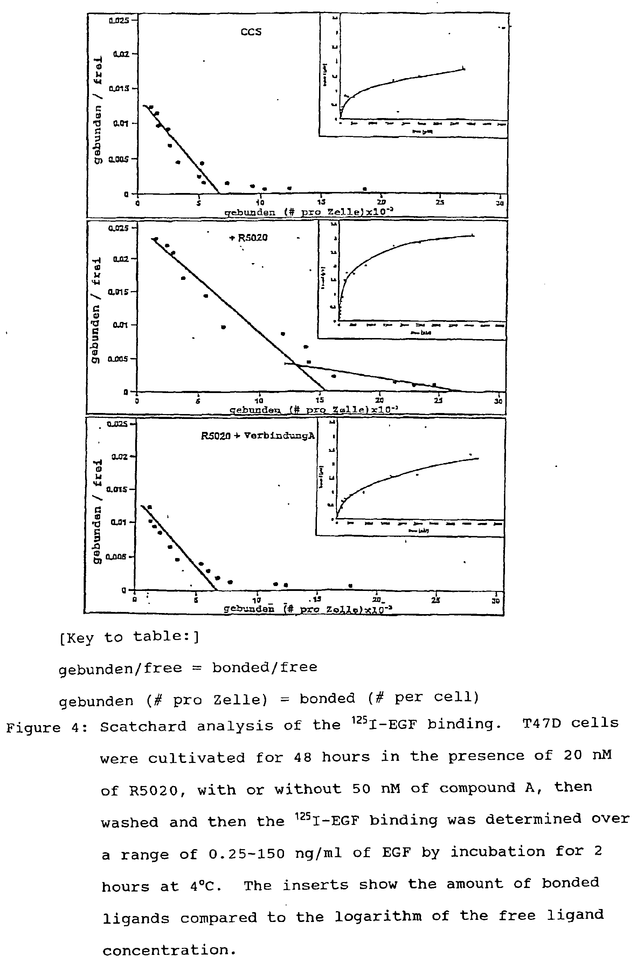

In Figure 4, a Scatchard analysis of the ¹²⁵I-EGF binding to T47D cells is shown. The cells were cultivated for 48 hours in the presence of 20 nM of R5020 with or without 20 nM of compound A and then washed. Then, the EGF-binding over a concentration range of 0.25 to 150 ng/ml of EGF was determined by incubation for 2 hours at 4°C. The insertions show the amount of bonded ligands relative to the logarithm of the free ligand concentration. It is clear that it was possible to block the increase of the EGF binding that is caused by R5020 (middle figure) relative to monitoring (upper figure) when compound A (lower figure) is added.

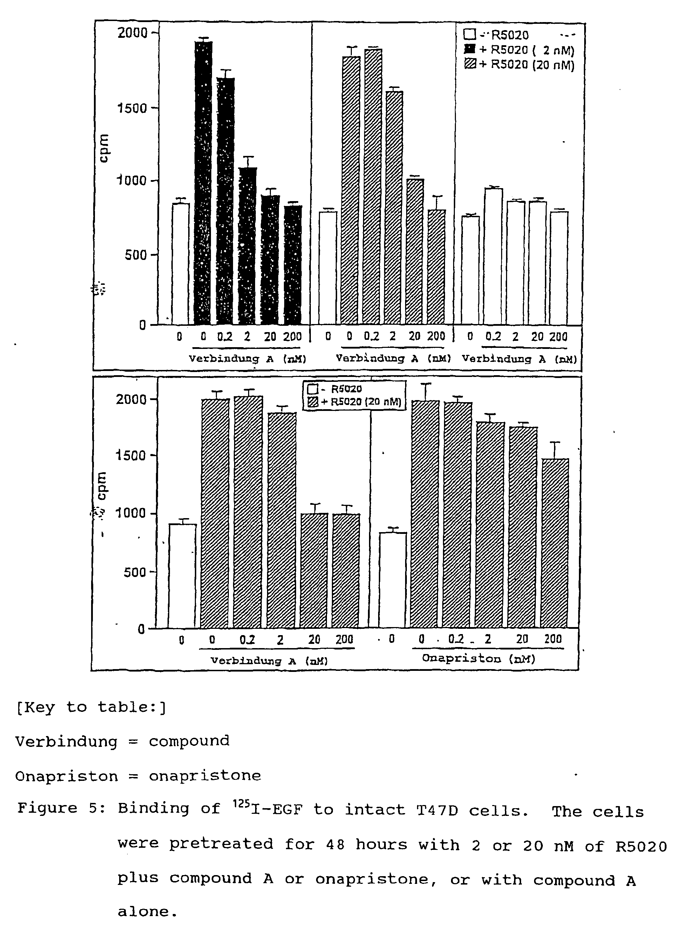

In figure 5, the binding of ¹²⁵I-EGF to intact T47D cells is shown. For this purpose, the cells were treated for 48 hours with 2 or 20 nM of R5020 plus compound A or onapristone or compound A alone. It can also be seen here that compound A blocks the increase of the EGF-binding to T47D cells caused by R5020. A similar -- although considerably weaker effect -- is also found for onapristone.

### 3. Discussion

The above results show that the estradiol-stimulated growth of T47D cells with high and constitutive PR contact was effectively blocked by compound A.

By transactivation assays, it was possible to show that the PR was transcriptionally active in the T47D cells and could be blocked by compound 1.

A stimulation of the T47D cells with R5020 resulted in a 2x to 3x-increased EGF-receptor expression, which was blocked by compound A. At the same time, the binding of EGF to the cells was increased 2- to 3-fold and could be prevented by compound A and less efficiently by onapristone. The increased EGF-binding to R5020-treated cells could be produced by an enhanced EGF-receptor expression or increased heterodimer formation between the EGF receptor and erbB2.

These results show the interactions between PR- and growth factor-signal systems in human breast carcinoma cells. By use of antiprogestins, the progression of tumor cells from steroid-dependent growth is inhibited or prevented for growth-factor-dependent growth.

## Claims

1. Use of a compound of general formula (I) 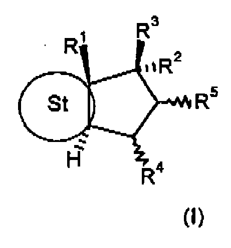 in which
R¹ stands for a methyl or ethyl group,
R² stands for a radical of formula CₙFₘHₒ, whereby n is 2, 3, 4, 5 or 6, m > 1 and m + 0 = 2n + 1,
R³ stands for a free, etherified, or esterified hydroxy group,
R⁴ and R⁵ each stand for a hydrogen atom, together for an additional bond or a methylene group,
St stands for a steroidal ABC-ring system of partial formula A, B or C 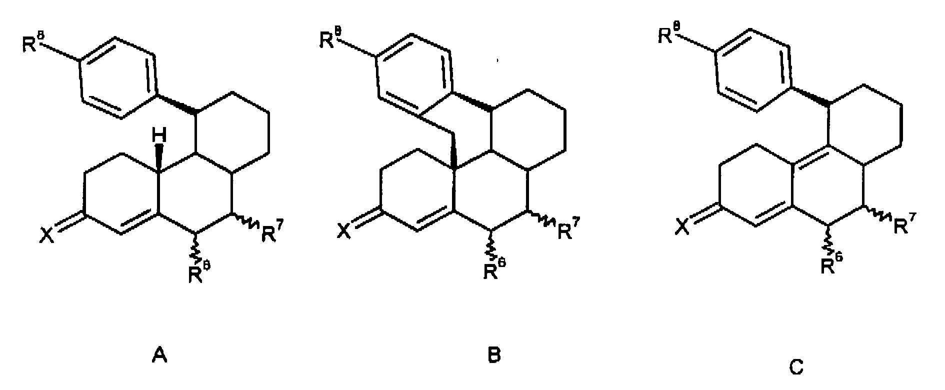
in which
R⁸ means a hydrogen atom, a straight-chain C₁-C₄ alkyl group or a branched C₃-C₄ alkyl group or a halogen atom,
R⁷ means a hydrogen atom, a straight-chain C₁-C₄ alkyl group or a branched C₃-C₄ alkyl group, or, if St stands for a steroidal ABC-ring system A or B, in addition R⁸ and R⁷ together mean an additional bond,
X means an oxygen atom, a hydroxyamino grouping = N-OH or two hydrogen atoms,
R⁸ means a radical Y or an aryl radical that is optionally substituted with a group Y in several places, whereby Y is a hydrogen atom, a halogen atom, an -OH, -NO₂, -N₃, -CN, -NR^{9a}R^{9b}, -NHSO₂R⁹, -CO₂R⁹, C₁-C₁₀ alkoxy, C₁-C₁₀ alkanoyloxy, benzoyloxy-C₁-C₁₀ alkanoyl, C₁-C₁₀ hydroxyalkyl or benzoyl group, and
R^{9a} and R^{9b} are the same or different and like R⁹ represent a hydrogen atom or a C₁-C₁₀ alkyl group,
and for radicals -NR^{9a}R^{9b}, also their physiologically compatible salts with acids and for radicals -CO₂R⁹ with R⁹ in the meaning of hydrogen also their physiologically compatible salts with bases,
for the manufacture of a medicament for the treatment of steroid resistant breast cancer.

2. Use according to claim 1, wherein the compound is 11β-(4-acety)phenyl)-17β-hydroxy-17α-(1,1,2,2,2-pentafluoraethyl)-estra-4,9-dien-3-one.

3. Use according to claims 1 and 2, wherein the binding of the growth factor EGF to the breast carcinoma cells is inhibited in tumor cells.

## Patentansprüche

1. Verwendung einer Verbindungn der allgemeinen Formel (I) 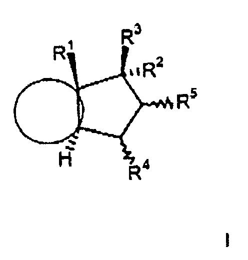 worin
R¹ für eine Methyl- oder Ethylgruppe,
R² für einen Rest der Formel CₙFₘHₒ, wobei n = 2, 3, 4, 5 oder 6, m > 1 und m + o = 2n + 1,
R³ für eine freie, veretherte oder veresterte Hydroxygruppe,
R⁴ und R⁵ je für ein Wasserstoffatom, gemeinsam für eine zusätzliche Bindung oder eine Methylengruppe,
St für ein steroidales ABC-Ringsystem der Teilformel A, B oder C 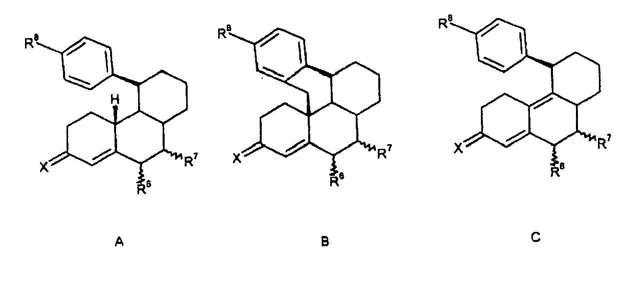
stehen,
worin
R⁶ ein Wasserstoffatom, eine geradkettige C₁-C₄₋oder verzweigte C₃-C₄-Alkylgruppe oder ein Halogenatom,
R⁷ ein Wasserstoffatom, eine geradkettige C₁-C₄₋oder eine verzweigte C₃-C₄-Alkylgruppe, oder, wenn St für ein steroidales ABC-Ringsystem A oder B steht, außerdem R⁶ und R⁷ gemeinsam eine zusätzliche Bindung,
X ein Sauerstoffatom, eine Hydroxyaminogruppierung =N-OH oder zwei Wasserstoffatome,
R⁸ einen Rest Y oder einen ggf. mehrfach mit einer Gruppe Y substituierten Arylrest, wobei Y ein Wasserstoffatom, ein Halogenatom, eine -OH, -NO₂, -N₃, -CN, -NR^{9a}R^{9b}, -NHSO₂R⁹, -CO₂R⁹, C₁-C₁₀₋Alkoxy-, C₁-C₁₀-Alkanoyloxy-, Benzoyloxy-C₁-C₁₀₋alkanoyl-, C₁-C₁₀-Hydroxyalkyl- oder Benzoylgruppe ist
und R^{9a} und R^{9b} gleich oder verschieden sind und wie R⁹ ein Wasserstoffatom oder eine C₁-C₁₀₋Alkylgruppe darstellen,
bedeuten,
sowie für die Reste -NR^{9a}R^{9b} auch deren physiologisch verträgliche Salze mit Säuren und für die Reste -CO₂R⁹ mit R⁹ in der Bedeutung von Wasserstoff auch deren physiologisch verträgliche Salze mit Basen zur Herstellung eines Medikaments zur Behandlung von steroidresistentem Brustkrebs.

2. Verwendung nach Anspruch 1, bei der die Verbindung 11β-(4-Acetylphenyl)-17β-hydroxy-17α-(1,1,2,2,2-pentafluorethyl)östra-4,9-dien-3-on ist.

3. Verwendung nach den Ansprüchen 1 und 2, bei der die Bindung des Wachstumsfaktors EGF an Mammakarzinomzellen in Tumorzellen gehemmt wird.

## Revendications

1. Utilisation d'un compose de formule générale (I) : 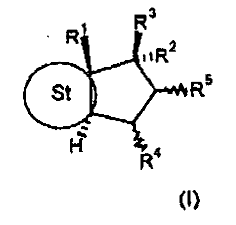 dans laquelle
R¹ correspond à un groupe méthyle ou éthyle,
R² correspond à un radical de formule CₙFₘHₒ, moyennant quoi n est 2, 3, 4, 5 ou 6, m > 1 et m + o = 2n + 1,
R³ correspond à un groupe hydroxyle libre, éthérifié ou estérifié,
R⁴ et R⁵ correspondent chacun à un atome d'hydrogène, ensemble pour une liaison supplémentaire, ou à un groupe méthylène,
St correspond à un système d'anneau ABC stéroïdien de formule partielle A, B ou C : 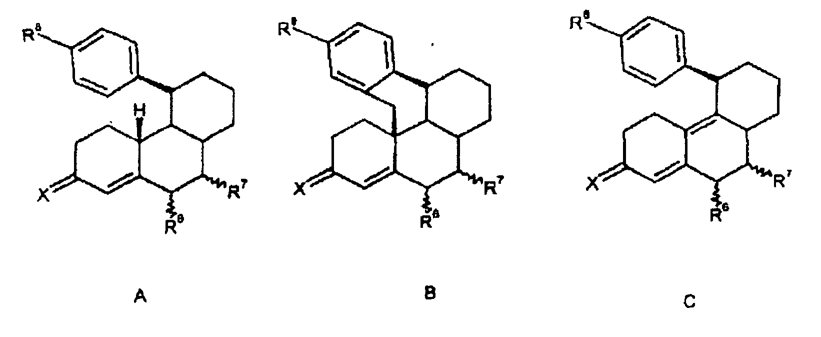
dans laquelle
R⁶ signifie un atome d'hydrogène, un groupe alkyle en C₁-C₄ à chaîne droite, un groupe alkyle en C₃-C₄ ramifié ou un atome d'halogène,
R⁷ signifie un atome d'hydrogène, un groupe alkyle en C₁-C₄ à chaîne droite, un groupe alkyle en C₃-C₄ ramifié ou bien, si le St correspond à un système A ou B d'un anneau ABC stéroïdien, R⁶ et R⁷ ensemble signifient en plus une liaison supplémentaire,
X signifie un atome d'oxygène, un regroupement d'hydroxyamino = N-OH ou deux atomes d'hydrogènes,
R⁸ signifie un radical Y ou un radical aryle qui est substitué facultativement par un groupe Y à plusieurs endroits, moyennant quoi le Y est : un atome d'hydrogène ; un atome d'halogène ; un -OH ; un -NO₂ ; un -N₃ ; un -CN ; un -NR^{9a}R^{9b} ; un -NHSO₂R⁹ ; un -CO₂R⁹ ; un alcoxy en C₁-C₁₀ ; un alcanoyloxy en C₁-C₁₀ ; un benzyloxy-alcanoyle en C₁-C₁₀ ; un hydroxyalkyle en C₁-C₁₀ ; ou un groupe benzoyle ; et
R^{9a} et R^{9b} sont identiques ou différents et, comme le R⁹, ils représentent un atome d'hydrogène ou un groupe alkyle en C₁-C₁₀
et, pour les radicaux -NR^{9a}R^{9b}, également leurs sels physiologiquement compatibles avec des acides
et, pour les radicaux -CO₂R⁹ avec un R⁹ signifiant un hydrogène, également leurs sels physiologiquement compatibles avec des bases,
pour la fabrication d'un médicament destiné au traitement d'un cancer du sein résistant aux stéroïdes.

2. Utilisation selon la revendication 1, dans laquelle le composé est la 11β-(4-acétylphényl)-17β-hydroxy-17α-(1,1,2,2,2-pentafluoroéthyl)-oestra-4,9-diène-3-one.

3. Utilisation selon les revendications 1 et 2, dans laquelle la liaison du facteur de croissance EGF aux cellules d'un carcinome du sein est inhibée dans les cellules tumorales.
